# EUROPEAN PATENT APPLICATION

(11) **EP 4 524 238 A1**
(43) Date of publication of application: **19.03.2025**
(21) Application number: 23803857.4
(22) Date of filing: 11.05.2023
(51) Int. Cl.: C12N 5/071, C12N 15/86, C12N 15/113, C12N 15/10, A61K 35/407, A61K 35/39, A61P 1/16, A61P 3/10

(54) **ENDOVASCULAR TRANSPLANT CELLS WITH HEMOCOMPATIBILITY, PREPARATION METHOD THEREFOR, AND USE THEREOF**

(30) Priority: 13.05.2022 KR 20220058776
(71) Applicant: Toolgen Incorporated, Seoul 07789 (KR)
(72) Inventor: LEE, Jae Young, Seoul 07789 (KR); AHN, Chang Hyun, Seoul 07789 (KR); LEE, Kang In, Seoul 07789 (KR); CHOI, Yu Ri, Seoul 07789 (KR)
(74) Representative: Modiano, Gabriella Diana
(86) International application number: PCT/KR2023/006394
(87) International publication number: WO 2023/219434

(57) **Abstract**

The present disclosure relates to cells for intravascular transplantation having hemocompatibility in which the blood coagulation mechanism and thrombosis reaction are inhibited by artificially modifying the F3 gene encoding CD142 that is an initiator of blood coagulation, using gene scissors technology to reduce or suppress the expression or activity of CD142, a method of producing the same, and a use thereof for preventing or treating liver disease and diabetes.

## Description

### TECHNICAL FIELD

The present disclosure provides cells for intravascular transplantation having hemocompatibility which may be administered intravascularly due to the inhibition of the thrombogenic reactions because of the reduction or inhibition of the expression or activity level of a blood coagulation initiating factor CD142, a preparation method therefor, and a use thereof.

### RELATED ART

Animals have a blood coagulation system, which is a complex biological mechanism that prevents bleeding to minimize blood loss due to tissue damage. The blood coagulation system is a continuous amplification response process called a cascade response in response to an initial stimulus, and is composed of an intrinsic pathway directly triggered by the stimulus and an independent extrinsic pathway. Many types of blood coagulation factors are involved here.

Among them, CD142 is a cell membrane glycoprotein that acts in the first step of the extrinsic pathway coagulation process and plays an important role in the in vivo coagulation process. After binding to coagulation factors VII or VIIa, CD142 generates thrombin during the coagulation process by activating coagulation factor X, which is involved in activating factor IX and prothrombin into thrombin, and thrombin activates fibrinogen into fibrin, forming thrombus. In addition, CD142 activates immune cells, causing the activation of neutrophils, platelets, and monocytes, triggering the secretion of various cytokines, and activating the inherent immune system.

Cell transplantation is a rapidly developing treatment in regenerative medicine, which is used for the purpose of treatment, diagnosis and prevention through a series of actions that isolate, proliferate and select cells, or change the biological characteristics of cells through the process of culturing living autologous, allogenic or xenegenic cells in an in vitro environment to restore the function of cells and tissues. Compared to whole organ transplantation, cell-based therapies are relatively less invasive procedures with lower morbidity/mortality. Cell transplantation has the advantage of significantly reducing the risks associated with surgery and allowing the original organ to still exist even when the graft function is insufficient or there is long-term graft loss.

Although pancreatic islet cell transplantation is suggested as the most ideal method for treating type 1 diabetes, there are matters in applying it to actual clinical practice due to limited survival of transplanted pancreatic islet cells caused by inflammatory responses such as instant blood-mediated inflammatory response (IBMIR). Portal vein thrombosis induced by pancreatic islet transplantation has become a well-known and feared complication. To prevent this, anticoagulants such as heparin are used. In instant blood-mediated inflammatory response, when transplanting pancreatic islet cells into the pancreatic portal vein, the transplanted pancreatic islet cells are directly exposed to blood, and blood coagulation occurs around the pancreatic islet cells due to activation of the blood coagulation system including platelets and complement, and the pancreatic islet cells are rapidly destroyed; and the inflammatory response is due to the dual activation of the blood coagulation and complement pathways by pancreatic islet cells (or cells) that possess CD142 (tissue factor (TF), blood coagulation factor III).

Hepatocyte transplantation is an alternative to liver transplantation for patients with certain liver-based metabolic diseases and acute liver failure. For transplantation, human hepatocytes are isolated from donor liver tissue using collagenase perfusion technology and purified using centrifugation. The number of transplanted cells is targeted to be approximately 5% of the normal liver mass and is usually administered through the hepatic portal vein or spleen. One of the major obstacles to the sustained success of this therapy is initial cell loss, with up to 70% of hepatocytes lost shortly after infusion. This is known to be mainly due to an instant blood-mediated inflammatory reaction (IBMIR). Transplanted hepatocytes produce and release CD142 (tissue factor (TF), blood coagulation factor III) that activates the coagulation pathway, forming thrombin and fibrin coagulation. Thrombin additionally activates multiple complement proteins, resulting in activation of the membrane attack complex (MAC) and subsequent hepatocyte death.

### SUMMARY

The present disclosure is for the purpose of providing cells for intravascular transplantation having hemocompatibility that inhibit a thrombogenic response that may be caused by the administered cells when administered intravascularly, a method of preparing the same, and a cell therapeutic agent using the same.

In order to achieve the above object, the present disclosure provides cells for intravascular transplantation having hemocompatibility that may be administered intravascularly by inhibiting the thrombogenic response by reducing or suppressing the expression or activity level of CD142, which is a blood coagulation initiating factor.

The present disclosure also provides artificially engineered mammalian cells for intravascular transplantation including an artificially engineered F3 gene, in which the artificially engineered F3 gene is different from the F3 gene sequence of wild-type mammalian cells, the artificially engineered F3 gene includes one or more indels in a nucleic acid sequence, and an expression level of CD142 of the artificially engineered mammalian cells is characterized as reduced compared to wild-type mammalian cells.

In addition, the present disclosure provides artificially engineered cells for intravascular transplantation, which are cells differentiated or derived from artificially engineered stem cells including an artificially engineered F3 gene, in which the artificially engineered F3 gene is different from the F3 gene sequence of cells differentiated or derived from wild-type stem cells, the artificially engineered F3 gene includes one or more indels in the nucleic acid sequence, and an expression level of CD142 on the surface of cells differentiated or derived from the artificially engineered stem cells is characterized as reduced compared to cells differentiated or derived from the wild-type stem cells.

In addition, the present disclosure provides a composition for preparing cells for intravascular transplantation having hemocompatibility, including a guide nucleic acid including a guide sequence capable of targeting a target sequence of an F3 gene of mammalian cells or stem cells; and an editor protein or a nucleic acid encoding the same.

In addition, the present disclosure provides a method of preparing cells for intravascular transplantation having hemocompatibility, including: (1) introducing a composition for preparing cells for intravascular transplantation having hemocompatibility into isolated mammalian cells or isolated stem cells; and (2) editing the F3 gene to reduce or suppress the expression or activity of CD142 by generating indels in the target sequence of the F3 gene located in the genome of the mammalian cells.

In addition, the present disclosure provides a cell therapeutic agent for vascular administration including, as an active ingredient, intravascular transplantation cells artificially engineered to have the hemocompatibility.

In addition, the present disclosure provides a pharmaceutical composition for preventing or treating liver disease, including, as an active ingredient, intravascular transplantation cells artificially engineered to have the hemocompatibility.

In addition, the present disclosure provides a pharmaceutical composition for preventing or treating diabetes, including, as an active ingredient, intravascular transplantation cells artificially engineered to have the hemocompatibility.

The artificially engineered intravascular transplantation cells of the present disclosure can inhibit the blood coagulation mechanism by reducing or suppressing the expression or activity of CD142 to artificially modify the F3 gene encoding CD142 that is a blood coagulation initiating factor, using genome editing technology. Therefore, the intravascular administration of transplant cells that were lost after transplantation due to inflammatory responses such as the instant blood-mediated inflammatory response (IBMIR) due to overexpression of CD142 on the cell surface and thus failed to exhibit a therapeutic effect can be made possible, and the loss of transplanted cells after transplantation can be prevented, thereby improving the therapeutic effect.

### BRIEF DESCRIPTION OF THE DRAWINGS

The above and other purposes, features and advantages of the present disclosure will become more apparent to those of ordinary skill in the art by describing embodiments thereof in detail with reference to the accompanying drawings, in which:

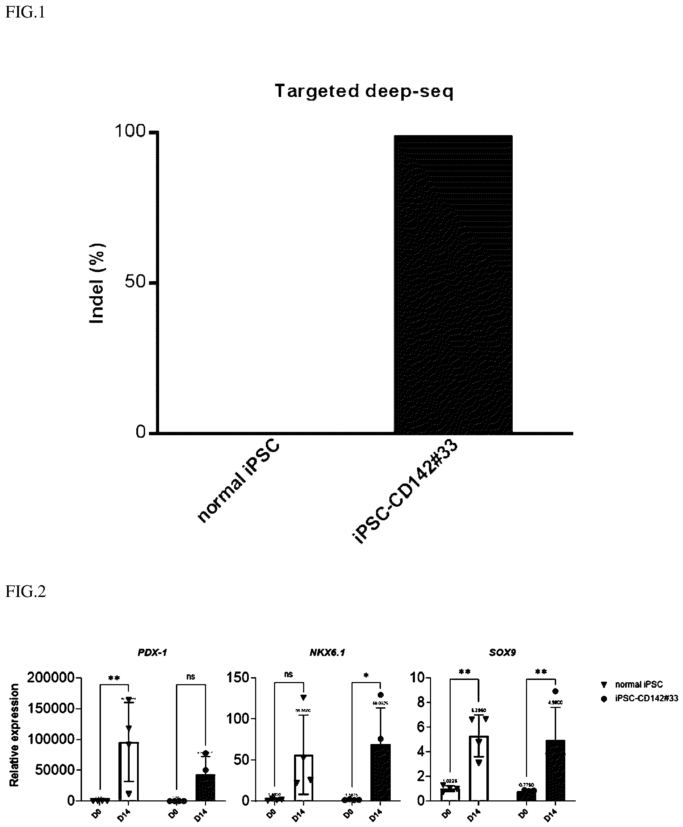

FIG. 1 is a graph showing the results of targeted deep sequencing of normal iPSCs and iPSCs that underwent gene editing of the target sequence (SEQ ID NO: 1) of CD142#33;
FIG. 2 shows the results of confirming the differentiation markers PDX-1, NKX6.1, and SOX9 of pancreatic lineage cells of the control group (normal iPSC), which is normal iPSC-derived pancreatic lineage cells, and the experimental group (iPSC-CD142#33), which is iPSC-derived pancreatic lineage cells with knocked out CD142 gene, using qRT-PCR;

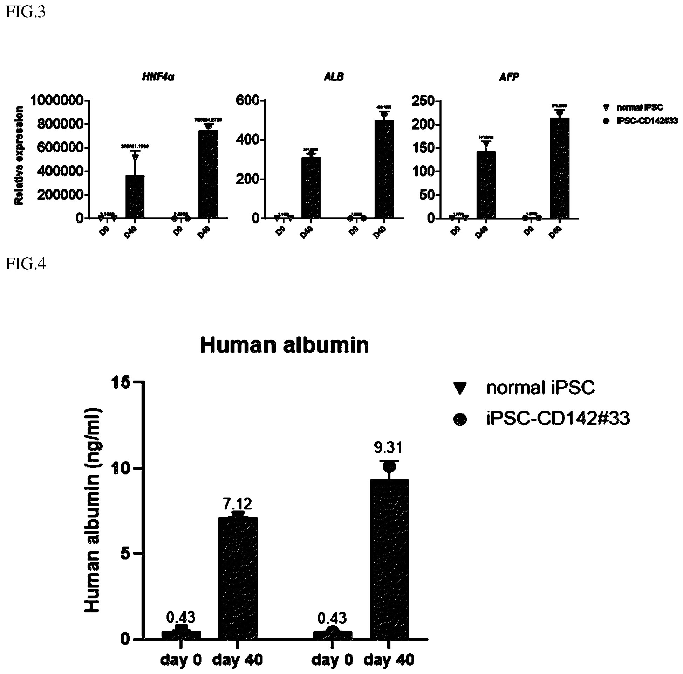

FIG. 3 shows the results of confirming the differentiation markers HNF4α, albumin (ALB), and AFP of liver cells of the control group (normal iPSC), which is normal iPSC-derived liver cells, and the experimental group (iPSC-CD142#33), which is iPSC-derived liver cells with knocked out CD142 gene, using qRT-PCR;
FIG. 4 shows the results of analyzing the differentiation marker ALB (albumin) of liver cells of the control group (normal iPSC), which is normal iPSC-derived liver cells, and the experimental group (iPSC-CD142#33), which is iPSC-derived liver cells with knocked out CD142 gene, using ELISA;

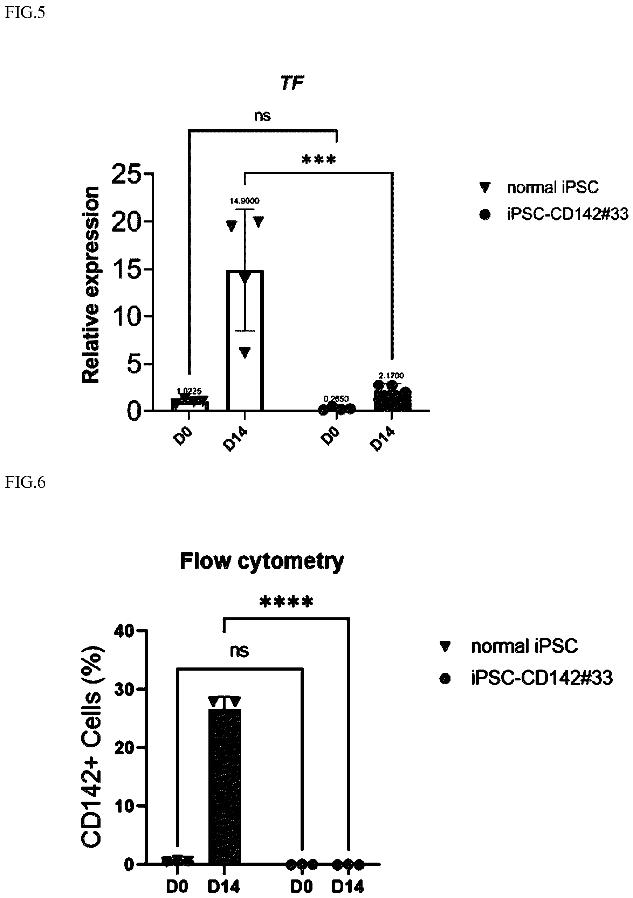

FIG. 5 shows the results of confirming the CD142 mRNA levels of the control group (normal iPSC), which is normal iPSC-derived pancreatic lineage cells, and the experimental group (iPSC-CD142#33), which is iPSC-derived pancreatic lineage cells with knocked out CD142 gene, using qRT-PCR;
FIG. 6 is a graph showing the results of measuring the percentage of cells showing CD142 expression (CD142 positive cell population, %) of the control group (normal iPSC), which is normal iPSC-derived pancreatic lineage cells, and the experimental group (iPSC-CD142#33), which is iPSC-derived pancreatic lineage cells with knocked out CD142 gene, using FACS;

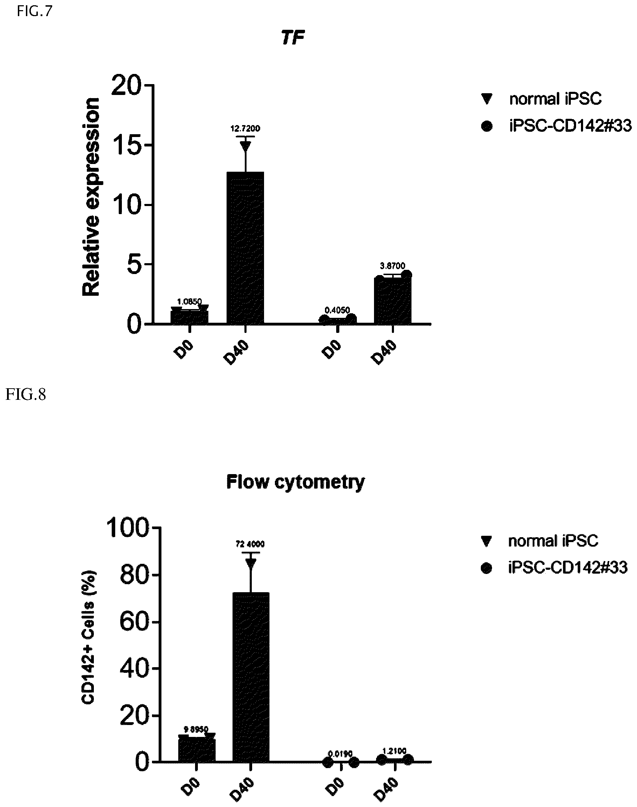

FIG. 7 shows the results of confirming the CD142 mRNA levels of the control group (normal iPSC), which is normal iPSC-derived liver cells, and the experimental group (iPSC-CD142#33), which is iPSC-derived liver cells with knocked out CD142 gene, using qRT-PCR;
FIG. 8 is a graph showing the results of measuring the percentage of cells showing CD142 expression (CD142 positive cell population, %) of the control group (normal iPSC), which is normal iPSC-derived liver cells, and the experimental group (iPSC-CD142#33), which is iPSC-derived liver cells with knocked out CD142 gene, using FACS;

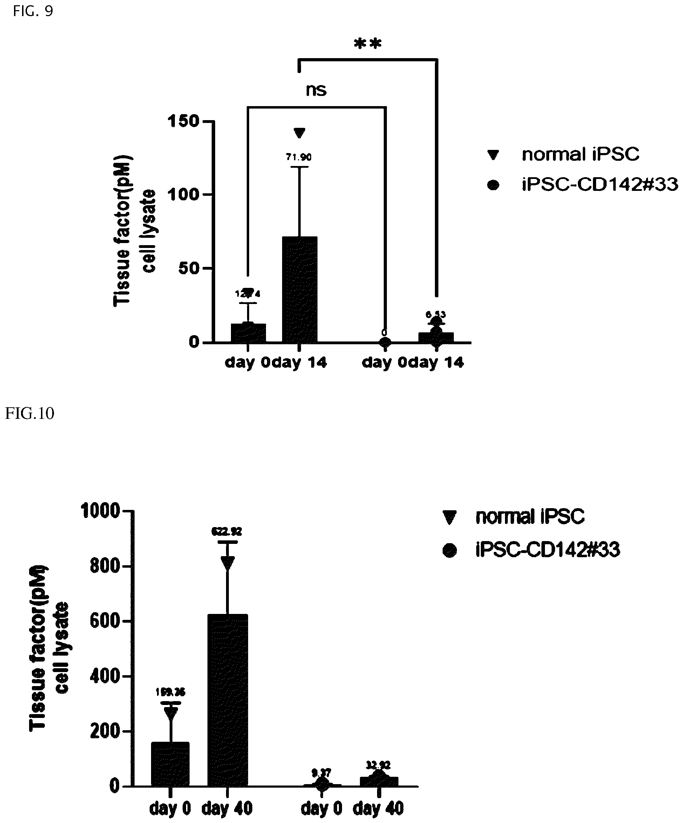

FIG. 9 shows the results of analyzing the amount of CD142 protein in cell lysates of the control group (normal iPSC), which is normal iPSC-derived pancreatic lineage cells, and the experimental group (iPSC-CD142#33), which is iPSC-derived pancreatic lineage cells with knocked out CD142 gene, using ELISA; and
FIG. 10 shows the results of analyzing the amount of CD142 protein in cell lysates of the control group (normal iPSC), which is normal iPSC-derived liver cells, and the experimental group (iPSC-CD142#33), which is iPSC-derived liver cells with knocked out CD142 gene, using ELISA.

### DETAILED DESCRIPTION

Hereinafter, the present disclosure will be described in more detail.

As used herein, the term "stem cell" refers to a broad concept that collectively refers to undifferentiated cells with stemness, that is, the ability to differentiate into various types of body tissue cells. At this time, the stem cells may be induced pluripotent stem cells, embryonic stem cells, somatic cell nuclear transfer-PSC, or adult stem cells. In addition, the cells may be of human origin, but are not limited thereto.

As used herein, "differentiation" refers to a phenomenon in which cells divide and proliferate and become specialized in their structure or function while the entire organism grows. In other words, the differentiation refers to a process by which cells and tissues of living things change into appropriate forms and functions to perform their assigned roles, and may include, for example, the process by which pluripotent stem cells change into ectoderm (cerebral cortex, midbrain, hypothalamus, etc.), mesoderm (yolk sac, etc.), and endoderm cells, as well as the process by which hematopoietic stem cells change into red blood cells, white blood cells, platelets, and the like, namely, the process by which progenitor cells express specific differentiation traits.

As used herein, the term "about" refers to quantity, level, value, number, frequency, percentage, dimension, size, amount, weight, or length that vary by 30, 25, 20, 25, 10, 9, 8, 7, 6, 5, 4, 3, 2 or 1 degrees with respect to a reference quantity, level, value, number, frequency, percentage, dimension, size, amount, weight, or length.

As used herein, the term "artificially engineered" is a term used to distinguish substances, molecules and the like that have a composition that is already present in the natural world, and means that artificial modifications have been made to the substances, molecules and the like. For example, the expression "artificially engineered gene" refers to a gene in which artificial modifications have been made to the composition of genes that are present in the natural world. In addition, the above term includes all meanings that may be recognized by a person skilled in the art, and may be appropriately interpreted depending on the context.

As used herein, the term "wild-type" refers to that a gene including a naturally occurring base sequence and a protein expressed from the gene have normal functional characteristics. Wild-type genes have a form in which no natural or artificial mutations have occurred and are most frequently observed in the population. When the term "wild-type" is used herein in contrast to artificially engineered genes and/or artificially engineered cells, this may be interpreted to mean a gene including an artificially engineered gene and/or a homologous "non-artificially engineered" naturally occurring base sequence corresponding to the artificially engineered cell, and a cell having the same. In addition, the above term includes all meanings that may be recognized by a person skilled in the art, and may be appropriately interpreted depending on the context.

As used herein, the expression "knock-out" or "knocked out gene" means that a mutation or artificial modification occurs in a wild-type gene, and as a result, means that the protein expressed by the wild-type gene is not produced through transcription and/or translation processes. For example, cells including knocked-out gene A may be unable to express the mRNA and/or protein expressed by wild-type gene A. Cells including a knocked-out gene A may be one in which only one gene A present in the cell is knocked out, or two or more genes A may be knocked out. In addition, the above term includes all meanings that may be recognized by a person skilled in the art, and may be appropriately interpreted depending on the context.

As used herein, the expression "knock-down" or "knocked down gene" means that a mutation or artificial modification occurs in a wild-type gene, resulting in the expression of a material in a lower amount than the wild-type gene. For example, cells including knocked down gene A may express less mRNA than that expressed by the wild-type gene A. As another example, cells including knocked down gene A may express a smaller amount of protein than the protein expressed by the wild-type gene A. Cells in which gene A has been knocked down may have only one gene A present in the cells knocked down, or have two or more genes knocked down. In addition, the above term includes all meanings that may be recognized by a person skilled in the art, and may be appropriately interpreted depending on the context.

As used herein, "expression reduction" means showing a lower level of expression than the expression level of mRNA and/or protein measured in the wild-type. The reduction may be a reduction of about 5% or more, about 10% or more, about 15% or more, about 20% or more, about 30% or more, about 50% or more, about 60% or more, about 70% or more, or about 100% or more compared to cells without genetic modification or wild-type cells.

As used herein, the term "activity reduction" or "reduced activity" may mean a reduction in relative activity when measuring protein or enzyme activity. Specifically, "activity reduction" or "reduced activity" means lower levels of protein, or enzyme activity compared to given parental or wild-type cells.

As used herein, the term "hemocompatibility" means not causing haemolysis, platelet adhesion, platelet activation, fibrin formation through complement activation, thrombogenic response, or embolism upon contact with blood.

The present disclosure provides cells for intravascular transplantation in which the expression or activity of CD142 (tissue factor (TF), blood coagulation factor III) is reduced or suppressed.

Cells for intravascular transplantation with reduced or suppressed expression or activity of CD142 are cell membrane glycoproteins that act in the first step of an exogenous coagulation process, and are mammalian cells in which the blood coagulation mechanism is inhibited by reducing or suppressing the expression or activity of CD142, which plays an important role in the blood coagulation process in vivo, and when utilized as a cell therapeutic agent and administered through the intravascular route, mammalian cells with reduced or suppressed expression or activity of CD142 may exhibit hemocompatibility showing improved hemocompatibility over wild-type mammalian cells, mammalian cells artificially engineered by techniques in the related art, or CD142 low-expressing mammalian cells selected with antibodies, cell sorting devices, or magnetic beads.

Specifically, the present disclosure provides hemocompatible artificially engineered intravascular transplantation cells characterized by reduced blood coagulation response upon intravascular administration by artificially engineering the F3 gene of mammalian cells to reduce or inhibit the expression or activity of F3 mRNA and/or CD142.

Mammals of the present disclosure include, for example, primates such as humans, monkeys, rhesus monkeys, crab-eating macaques, marmosets, orangutans, and chimpanzees; rodents such as mice, rats, hamsters, and guinea pigs; lagomorphs such as rabbits; ungueatas such as pigs, cows, goats, horses, and sheep; and felines such as dogs and cats, and among these, mice, pigs, and humans are particularly preferable.

According to one embodiment of the present disclosure, the cells for transplantation of the present disclosure are allogenic or autologous cells for transplantation, and more specifically, are allogenic cells for transplantation.

According to one embodiment of the present disclosure, the mammalian cell of the present disclosure may be a primary hepatocyte, a primary pancreatic beta cell, a primary pancreatic islet cell, a pancreatic progenitor cell, a pluripotent stem cell (PSC)-derived hepatocyte, a PSC-derived pancreatic beta cell, a PSC-derived pancreatic islet cell, a PSC-derived pancreatic lineage cell, a PSC-derived pancreatic progenitor cell, a PSC-derived pancreatic organoid, a PSC-derived pancreatic liver organoid, an iPSC-derived hepatocyte, an iPSC-derived pancreatic beta cell, an iPSC-derived pancreatic islet cell, an iPSC-derived pancreatic lineage cell, an iPSC-derived pancreatic progenitor cell, an iPSC-derived pancreatic organoid, iPSC-derived pancreatic liver organoid, induced hepatocytes (iHeps) from somatic cells by a compound or gene manipulation, a pancreatic islet cell induced from somatic cells by a compound or gene manipulation or a pancreatic beta cell induced from somatic cells by a compound or gene manipulation.

According to one embodiment of the present disclosure, the cells for transplantation of the present disclosure are artificially engineered intravascular transplantation cells, which are cells differentiated or derived from artificially engineered stem cells including an artificially engineered F3 gene, wherein the artificially engineered F3 gene is different from the F3 gene sequence of cells differentiated or derived from wild-type stem cells, the artificially engineered F3 gene includes one or more indels in the nucleic acid sequence, and an expression level of CD142 on the surface of cells differentiated or derived from the artificially engineered stem cells is reduced compared to cells differentiated or derived from the wild-type stem cells.

According to one embodiment of the present disclosure, the stem cells are induced pluripotent stem cells, embryonic stem cells, somatic cell nuclear transfer-PSC, or adult stem cells.

According to one embodiment of the present disclosure, the cells differentiated or derived from the artificially engineered stem cells are pancreatic beta cells, hepatocytes, pancreatic progenitor cells, pancreatic islet cells, pancreatic lineage cells, pancreatic organoids, or pancreatic liver organoids.

The present disclosure provides artificially engineered cells for intravascular transplantation including an artificially modified nucleic acid sequence of the F3 gene.

In the present disclosure, "artificial modification" or "artificial engineering" of a gene nucleic acid sequence may be achieved through modification of the nucleic acid sequence constituting the gene or chemical modification of a single base. This may be due to mutation, substitution, or deletion of part or all of the genes, or the insertion of one or more bases into the gene, and may be achieved using gene editing technology such as the CRISPR-enzyme system. As an example, artificial modification of the gene nucleic acid sequence may be achieved by non-homologous end joining (NHEJ) or homology directed repair (HDR) mechanisms.

For example, the artificially engineered intravascular transplantation cells may have the F3 gene knocked out.

As used herein, "non-homologous end joining (NHEJ)" is a method of restoring or repairing a double-strand break in DNA by joining both ends of a cut double-strand or single strand together, and when two compatible ends, usually formed by a break in the double strand (e.g., cleavage), repeat frequent contacts such that the two ends become fully joined, broken double strands are repaired.

In the process of repairing damaged genes or nucleic acids using NHEJ, some "insertion and/or deletion" (or "indels", InDels) of nucleic acid sequences may occur at the NHEJ repair site, and the gene in which an indel occurred does not have the same sequence as the wild-type gene. This insertion and/or deletion changes the reading frame of the gene, produce frame shifted transcript mRNA, and eventually loses its original function by undergoing nonsense-mediated decay or failing to synthesize normal proteins. In addition, mutations may occur that maintain the reading frame but insert or delete significant amounts of sequence, destroying the functionality of the protein. As another example, when an indel occurs in a transcriptional regulatory region such as the promoter region or enhancer region of a gene, the mRNA may not be transcribed or the transcription amount may be reduced, and thus the protein may not be expressed or the expression amount may be reduced. Alternatively, the mutagenesis mechanism of NHEJ may be utilized to delete only some sequence motifs when the generation of a specific final sequence is not required. For example, when two or more guide RNAs targeting the 5' and 3' intron regions of a specific exon are used to cause a double-strand break in each intron, and only part of the exon of the gene is deleted by NHEJ, other portions can be expressed normally and the main functionality of the protein may be maintained.

Using this NHEJ, the gene of interest may be specifically knocked out or knocked down using genome editing technology.

For example, CRISPR enzymes such as Cas9 or Cpf1, a type of genetic scissors, are used to cut double strands or two single strands of the target gene or target nucleic acid, indels re generated by NHEJ on a double strand or two single strands of a broken target gene or a broken target nucleic acid, and through this, specific knock-out or knock-down of the target gene or nucleic acid may be induced.

For example, the artificially engineered cells for intravascular transplantation may have the F3 gene knocked down.

In one embodiment of the present disclosure, the F3 gene of the artificially engineered intravascular transplantation cells may include one or more indels in the nucleic acid sequence.

In one embodiment of the present disclosure, the artificially engineered intravascular transplantation cells may not express F3 mRNA.

In one embodiment of the present disclosure, the mRNA transcribed from the artificially engineered F3 gene of the artificially engineered intravascular transplantation cells may have lower expression levels of that mRNA compared to the level of mRNA transcribed from the F3 gene of wild-type cells.

In one embodiment of the present disclosure, the artificially engineered intravascular transplantation cells may have different F3 mRNA sequences compared to wild-type cells.

In one embodiment of the present disclosure, the artificially engineered intravascular transplantation cells may have reduced expression or activity of CD142 on the cell surface compared to wild-type cells. Through this, the function of the artificially engineered intravascular transplantation cells of the present disclosure may be reduced or lost.

That is, in the artificially engineered intravascular transplantation cells, the expression or activity of CD142 may be reduced by about 20% or more, about 30% or more, about 40% or more, about 50% or more, about 55% or more, about 60% or more, about 70% or more, about 75% or more, about 80% or more, about 85% or more, about 90% or more, about 95% or more, or about 100% compared to the expression or activity of wild-type cells.

The present disclosure provides a method of producing hemocompatible artificially engineered intravascular transplantation cells, including reducing the expression or activity of CD142 of the mammalian cells.

The increase or decrease in the expression or activity of CD142 may be achieved by artificial modification of the F3 gene, for example, using gene editing technology.

As an example, the genome editing technology may utilize TALEN (transcription activator like effctor nuclease) in which a transcription activator-like (TAL) effector (TALE) domain and a cutting domain are fused, a zinc-finger nuclease, or CRISPR-enzyme system derived from a clustered regularly interspaced short palindromic repeats (CRISPR), which is a microbial immune system, but is not limited thereto.

The entire contents disclosed in International Patent Publication No. WO2012/093833 or U.S. Patent Publication No. 2013-0217131 with respect to the above TALEN are incorporated in the present specification by reference. In relation to the above ZFN, Beerli et al. (2002) Nature Biotechnol. 20:135-141; Pabo et al. (2001) Ann. Rev. Biochem. 70:313-340; Isalan et al, (2001) Nature Biotechnol. 19: 656-660; Segal et al. (2001) Curr. Opin. Biotechnol. 12:632-637; Choo et al. (2000) Curr. Opin. Struct. Biol.10:411-416; and U.S. Patent Nos. 7,888,121, 8,409,861, 6,479,626, 6,903,185, and 7,153,949 may be included as reference material in the present specification.

The "CRISPR-enzyme system" consists of a guide nucleic acid and/or an editor protein.

"Guide nucleic acid" refers to a nucleic acid capable of recognizing a target nucleic acid, target gene, or target chromosome and interacting with an editor protein. At this time, the guide nucleic acid may form a complementary bond with some nucleotides in the target nucleic acid, target gene, or target chromosome.

The guide nucleic acid may be in the form of a target DNA-specific guide RNA, DNA encoding the guide RNA, or a DNA/RNA mixture.

The guide nucleic acid may be a guide RNA. As an example, the "guide RNA" may be transcribed in vitro, particularly from an oligonucleotide double strand, or a plasmid template. As another example, the guide RNA may be encoded in the form of a vector, and may be transferred into a cell in an ex vivo or in vivo environment and transcribed from the vector, but is not limited thereto.

The design and composition of the guide RNA are known to those skilled in the art and are described in detail in Korean Registration Patents 10-1656236, 10-1656237, 10-1706085, 10-2052286, and 10-2182847, and the entirety of the above Registration Patents is incorporated herein as reference material for the present disclosure.

The guide nucleic acid may include a scaffold sequence portion and a guide sequence portion. The scaffold sequence portion is a portion that interacts with the Cas protein and allows the Cas protein and a guide nucleic acid to bind to form a complex (ribonucleoprotein, RNP). Generally, the scaffold sequence portion includes tracrRNA and some sequence portions of crRNA, and the scaffold sequence is determined depending on which Cas protein is used.

The guide sequence portion is a nucleotide sequence portion that may bind complementary to some sequences of any one of the double strands of a target gene or nucleic acid, and a nucleotide sequence portion that may be artificially modified, and is determined by the target nucleotide sequence of interest. At this time, the guide sequence may be a nucleotide sequence having at least 50%, 55%, 60%, 65%, 70%, 75%, 80%, 85%, 90%, or 95% or more complementarity or complete complementarity with the guide nucleic acid binding sequence of the target gene or target nucleic acid. The guide sequence may be a sequence included in the guide domain of the guide nucleic acid.

The guide sequence portion may be included in crRNA. As an example, the guide nucleic acid may be a dual RNA including two RNAs, namely, crRNA (CRISPR RNA) and tracrRNA (trans-activating crRNA) as components.

As another example, the guide nucleic acid may be a single-chain guide RNA (sgRNA) in which the main portions of crRNA and tracrRNA are linked.

The target sequence may be a nucleotide sequence of a certain length present in the target gene or target nucleic acid, and specifically, some nucleotide sequences within the target region classified by a regulatory region of the target gene, coding region (or CDS, coding sequence), or a non-coding region (or untranslated region (UTR)), or one or more some nucleotide sequences selected from a combination of the target regions. The target sequence may be a target of a guide nucleic acid-editor protein complex (RNP).

In one embodiment of the present disclosure, the target sequence may be a sequence included in a first region, second region, third region, fourth region, or sixth region of the exon of the wild-type F3 gene.

In one embodiment of the present disclosure, the target sequence may be the sequence of SEQ ID NO: 1.

The target sequence is a nucleotide sequence adjacent to a protospacer-adjacent motif (PAM) sequence recognized by the editor protein, and may include all or part of the PAM, but is not limited thereto.

The term "target sequence" may be used to mean both types of nucleotide sequence information. For example, in the case of a target gene, the target sequence may mean sequence information of the transcribed strand of the target gene DNA, or may mean nucleotide sequence information of the non-transcribed strand.

The target sequence includes a guide nucleic acid binding sequence or a guide nucleic acid-nonbinding sequence. The guide nucleic acid binding sequence is a nucleotide sequence that has partial or complete complementarity with the guide sequence included in the guide domain of the guide nucleic acid, and may bind complementary to the guide sequence included in the guide domain of the guide nucleic acid. The target sequence and guide nucleic acid binding sequence are nucleotide sequences that may vary depending on the target gene or nucleic acid, that is, depending on the subject to be genetically engineered or corrected, and the guide nucleic acid may be designed in various ways depending on the target gene or target nucleic acid.

The guide nucleic acid non-binding sequence is a nucleotide sequence that has partial or complete complementarity with the guide sequence included in the guide domain of the guide nucleic acid, and cannot bind complementary to the guide sequence included in the guide domain of the guide nucleic acid. In addition, the guide nucleic acid non-binding sequence is a nucleotide sequence that is complementary to the guide nucleic acid binding sequence and may bind complementary to the guide nucleic acid binding sequence. The guide nucleic acid binding sequence is some nucleotide sequences of the target sequence, and may be a nucleotide sequence having two different sequence orders of the target sequence, that is, one nucleotide sequence of two nucleotide sequences capable of complementary binding to each other. At this time, the guide nucleic acid non-binding sequence may be the remaining nucleotide sequence of the target sequence excluding the guide nucleic acid binding sequence.

The guide nucleic acid binding sequence may be a target sequence, that is, one nucleotide sequence selected from a nucleotide sequence identical to the transcribed strand and a nucleotide sequence identical to the non-transcribed strand. At this time, the guide nucleic acid non-binding sequence may be the guide nucleic acid binding sequence among the target sequences, that is, the remaining nucleotide sequence excluding one nucleotide sequence selected from the nucleotide sequence identical to the transcribed strand and the nucleotide sequence identical to the non-transcribed strand.

The guide nucleic acid binding sequence may be the same length as the target sequence. The guidenucleic acid non-binding sequence may be the same length as the target sequence or guidenucleic acid binding sequence. The guide nucleic acid binding sequence may be 5 to 50 nucleotide sequences.

In one embodiment, the guide nucleic acid binding sequence is 16 nucleotide sequences, 17 nucleotide sequences, 18 nucleotide sequences, 19 nucleotide sequences, 20 nucleotide sequences, 21 nucleotide sequences, 22 nucleotide sequences, 23 nucleotide sequences, 24 nucleotide sequences, or 25 nucleotide sequences. The guide nucleic acid non-binding sequence may be 5 to 50 nucleotide sequences.

In one embodiment, the guide nucleic acid non-binding sequence is 16 nucleotide sequences, 17 nucleotide sequences, 18 nucleotide sequences, 19 nucleotide sequences, 20 nucleotide sequences, 21 nucleotide sequences, 22 nucleotide sequences, 23 nucleotide sequences, 24 nucleotide sequences, or 25 nucleotide sequences.

The guide nucleic acid binding sequence may form a partial or complete complementary bond with the guide sequence included in the guide domain of the guide nucleic acid, and the length of the guide nucleic acid binding sequence may be the same as the length of the guide sequence.

The guide nucleic acid binding sequence may be a nucleotide sequence complementary to the guide sequence included in the guide domain of the guide nucleic acid, for example, a nucleotide sequence that is at least 70%, 75%, 80%, 85%, 90%, or 95% complementary or completely complementary.

As an example, the guide nucleic acid binding sequence may have or include a 1 to 8 nucleotide sequences that is not complementary to the guide sequence included in the guide domain of the guide nucleic acid.

The guide nucleic acid non-binding sequence may have partial or complete homology to the guide sequence included in the guide domain of the guide nucleic acid, and the length of the guide nucleic acid non-binding sequence may be the same as the length of the guide sequence. As an example, the guide sequence may be designed based on a sequence having homology to the guide nucleic acid non-binding sequence.

The guide nucleic acid non-binding sequence may be a nucleotide sequence having homology to the guide sequence included in the guide domain of the guide nucleic acid, for example, a nucleotide sequence having at least 70%, 75%, 80%, 85%, 90%, or 95% homology or completely homology.

As an example, the guide nucleic acid non-binding sequence may have or include a 1 to 8 nucleotide sequences that is not homologous to the guide sequence included in the guide domain of the guide nucleic acid. The guide nucleic acid non-binding sequence may bind complementary to the guide nucleic acid binding sequence, and the guide nucleic acid non-binding sequence may be the same as the length of the guide nucleic acid binding sequence.

The guide nucleic acid non-binding sequence may be a nucleotide sequence complementary to the guide nucleic acid binding sequence, for example, a nucleotide sequence that is at least 90% or 95% complementary or completely complementary.

As an example, the guide nucleic acid non-binding sequence may have or include 1 to 2 nucleotide sequences that are not complementary to the guide nucleic acid binding sequence. In addition, the guide nucleic acid binding sequence may be a nucleotide sequence located close to a complementary sequence complementary to a nucleotide sequence (PAM sequence) that may be recognized by the editor protein.

As an example, the guide nucleic acid binding sequence may be a contiguous 5 to 50 nucleotide sequences located adjacent to the 5' end or/and 3' end of a sequence complementary to a nucleotide sequence (PAM sequence) that may be recognized by the editor protein.

In addition, the guide nucleic acid non-binding sequence may be a nucleotide sequence located close to a nucleotide sequence (PAM sequence) that may be recognized by the editor protein.

As an example, the guide nucleic acid non-binding sequence may be a contiguous 5 to 50 nucleotide sequences located adjacent to the 5' end or/and 3' end of a nucleotide sequence (PAM sequence) that may be recognized by the editor protein.

"Editor protein" means a peptide, polypeptide or protein that either bind directly to nucleic acids, or do not bind directly, but may interact a nucleic acid. The editor protein is conceptually referred to as "artificially engineered nuclease" or RNA-guided endonuclease RGEN).

In one embodiment, the editor protein may be a CRISPR enzyme. "CRISPR enzyme" is the main protein component of the CRISPR-enzyme system, also called "Cas protein (CRISPR associated protein)", and refers to a nuclease that may recognize a target sequence and cleave DNA by mixing or forming a complex with a guide RNA.

CRISPR enzymes are known to those skilled in the art, see Korean Registration Patents Nos. 10-1656236, 10-1656237, 10-1706085, 10-2052286, and 10-2182847. The CRISPR enzyme is used herein as a concept that includes all variants that may act as an endonuclease or nickase activated in cooperation with a guide RNA, in addition to the native protein. In the case of activated endonuclease or nickase, target DNA may be cut and this may be used to perform genome editing. In addition, in the case of an inactivated variant, the genome editing may be used to regulate transcription or isolate the desired DNA.

The CRISPR enzyme is a nucleic acid or polypeptide (or protein) having a sequence encoding the CRISPR enzyme, typically, Type II CRISPR enzyme or Type V CRISPR enzyme is widely used, and the Type II CRISPR enzyme is CRISPR associated protein 9 (Cas9) protein.

The Cas9 protein may be derived from various microorganisms such as *Streptococcus pyogenes, Streptococcus thermophilus, Streptococcus* sp., *Campylobacter jejuni, Staphylococcus aureus, Staphylococcus Auricularis,* and *Neisseria meningitidis.*

In order for the Cas9 protein to induce a double-stranded DNA break, the Cas9 protein recognizes a protospacer-adjacent motif (PAM) sequence, which is a nucleotide sequence of a certain length, and requires that a portion of the guide RNA (the guide sequence portion) bind complementary to the complementary strand (the guide nucleic acid binding sequence) of a single strand of DNA (the guide nucleic acid non-binding sequence) on which the target sequence is located.

This PAM sequence is a sequence determined depending on the type or origin of the Cas9 protein, for example, a *Streptococcus pyogenes-*derived Cas9 protein (SpCas9) may recognize the 5'-NGG-3' sequence (complementary sequence: 5'-CCN-3') in the target nucleic acid. At this time, N is one of adenosine (A), thymidine (T), cytidine (C), and guanosine (G). In addition, SpCas9 can recognize the 5'-NAG-3' sequence (complementary sequence: 5'-CTN-3') in the target nucleic acid with low activity.

In addition, the Type V CRISPR enzyme includes Cpf1, and Cpf1 may be *Streptococcus, Campylobacter, Nitratifractor, Staphylococcus, Parvibaculum, Roseburia, Neisseria, Gluconacetobacter, Azospirillum, Sphaerochaeta,Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, uberibacillus, Bacillus, Brevibacilus, Methylobacterium,* or *Acidaminococcus-derived* Cpf1.

CRISPR enzymes such as the Cas9 or Cpf1 protein may be isolated from microorganisms present in nature or produced unnaturally through recombinant or synthetic methods. The Cas protein may also be in a form that is easy to introduce into cells. For example, the Cas protein may be linked to a cell-penetrating peptide or protein transduction domain. The protein transduction domain may be poly-arginine or HIV-derived TAT protein, but is not limited thereto. Since various types of cell-penetrating peptides or protein transduction domains are known in the art in addition to the examples described above, those skilled in the art are not limited to the above examples and may apply various examples to the present specification. In addition, the Cas protein may be fused with a functional domain such as a nuclear localization sequence or signal (NLS). In addition, the Cas9 protein may be encoded in the form of a vector and expressed within cells.

The present disclosure provides a composition for preparing cells for intravascular transplantation having hemocompatibility, including a guide nucleic acid including a guide sequence capable of targeting a target sequence of an F3 gene of mammalian cells or stem cells; and an editor protein or a nucleic acid encoding the same.

In addition, the composition may optionally further include a donor including a specific nucleotide sequence to be inserted or a nucleic acid encoding the same.

The donor refers to exogenous nucleotide sequences that may express a specific peptide or protein, and may be inserted into genomic DNA through homology directed repair (HDR).

The donor may be a double-stranded nucleic acid or a single-stranded nucleic acid. The donor may be linear or circular.

The donor may be in the form of a viral vector or a non-viral vector (e.g., a plasmid).

The virus may be a DNA virus or an RNA virus. At this time, the DNA virus may be a double-stranded DNA (dsDNA) virus or a single-stranded DNA (ssDNA) virus. At this time, the RNA virus may be a single-stranded RNA (ssRNA) virus.

The viral vector may be one or more viral vectors selected from the group consisting of retrovirus, lentivirus, adenovirus, adeno-associated virus (AAV), vaccinia virus, poxvirus, and herpes simplex virus (HSV).

The target sequence may be a target of a guide nucleic acid-editor protein complex, and the target sequence may include a protospacer-adjacent motif (PAM) sequence recognized by the editor protein, but is not limited thereto.

The guide nucleic acid may include a guide domain capable of targeting the target sequence of the F3 gene.

In the present specification, the guide nucleic acid, editor protein or guide nucleic acid-editor protein complex (ribonucleoprotein, RNP) and/or donor may be delivered or introduced into the subject in various forms.

In this case, the "subject" refers to an organism into which the guide nucleic acid, editor protein, or guide nucleic acid-editor protein complex is introduced; an organism in which a guide nucleic acid, an editor protein, or a guide nucleic acid-editor protein complex operates; or a specimen or sample obtained from the organism.

The subject may be an organism including a target gene, a target nucleic acid, or a target chromosome of the guide nucleic acid-editor protein complex.

The organism may be an animal, animal tissue, or animal cell. At this time, the tissue may be the eye, skin, liver, kidney, heart, lung, brain, muscle, or blood.

The cells may be mammalian cells.

Mammals of the present disclosure include, for example, primates such as humans, monkeys, rhesus monkeys, crab-eating macaques, marmosets, orangutans, and chimpanzees; rodents such as mice, rats, hamsters, and guinea pigs; lagomorphs such as rabbits; ungueatas such as pigs, cows, goats, horses, and sheep; and felines such as dogs and cats, and among these, mice, pigs, and humans are particularly preferable.

According to one embodiment of the present disclosure, the artificially engineered intravascular transplantation cells of the present disclosure are allogenic or autologous cells for transplantation, and more specifically, are allogenic cells for transplantation.

According to one embodiment of the present disclosure, the mammalian cell of the present disclosure may be a primary hepatocyte, a primary pancreatic beta cell, a primary pancreatic islet cell, a pancreatic progenitor cell, a pluripotent stem cell (PSC)-derived hepatocyte, a PSC-derived pancreatic beta cell, a PSC-derived pancreatic islet cell, a PSC-derived pancreatic lineage cell, a PSC-derived pancreatic progenitor cell, a PSC-derived pancreatic organoid, a PSC-derived pancreatic liver organoid, an iPSC-derived hepatocyte, an iPSC-derived pancreatic beta cell, an iPSC-derived pancreatic islet cell, an iPSC-derived pancreatic lineage cell, an iPSC-derived pancreatic progenitor cell, an iPSC-derived pancreatic organoid, iPSC-derived pancreatic liver organoid, induced hepatocytes (iHeps) from somatic cells by a compound or gene manipulation, a pancreatic islet cell induced from somatic cells by a compound or gene manipulation or a pancreatic beta cell induced from somatic cells by a compound or gene manipulation.

The specimen or sample may be acquired from an organism including target gene, target nucleic acid, or target chromosome, such as saliva, blood, liver tissues, brain tissues, liver cells, nerve cells, phagocytes, macrophages, T cells, B cells, astrocytes, cancer cells, or stem cells.

The guide nucleic acid, editor protein, or guide nucleic acid-editor protein complex may be delivered or introduced into the subject in the form of DNA, RNA, or a mixture thereof.

At this time, DNA, RNA, or a mixture thereof encoding the guide nucleic acid and/or the editor protein may be delivered or introduced into the subject by methods known in the art.

Alternatively, the form of DNA, RNA, or a mixture thereof encoding the guide nucleic acid and/or the editor protein may be delivered or introduced into the subject by vector, non-vector, or a combination thereof.

The vector may be a viral vector or a non-viral vector (e.g., a plasmid).

The virus may be a DNA virus or an RNA virus. At this time, the DNA virus may be a double-stranded DNA (dsDNA) virus or a single-stranded DNA (ssDNA) virus. At this time, the RNA virus may be a single-stranded RNA (ssRNA) virus.

The viral vector may be one or more selected from the group consisting of retrovirus, lentivirus, adenovirus, adeno-associated virus (AAV), vaccinia virus, poxvirus, and herpes simplex virus.

The non-vector may be naked DNA, DNA complex, or mRNA.

In one embodiment of the present disclosure, the nucleic acid encoding the guide nucleic acid and/or the editor protein may be delivered or introduced into the subject in the form of one or more vectors.

The vector may include a guide nucleic acid and/or a nucleic acid encoding an editor protein. As an example, the vector may simultaneously include a guide nucleic acid and a nucleic acid encoding an editor protein. As another example, the vector may include a nucleic acid encoding a guide nucleic acid. For example, the nucleic acid encoding the guide nucleic acid may be all included in one vector, or the nucleic acid encoding the guide nucleic acid may be divided and included in multiple vectors. As another example, the vector may include a nucleic acid encoding an editor protein. For example, in the case of the editor protein, the nucleic acid encoding the editor protein may be included in one vector, or the nucleic acid encoding the editor protein may be divided and included in multiple vectors.

The editor protein may be delivered or introduced into the subject in the form of peptide, polypeptide, or protein.

The editor protein may be delivered or introduced into the subject in the form of peptide, polypeptide, or protein by methods known in the art.

The guide nucleic acid and the editor protein may be delivered or introduced into the subject in the form of a nucleic acid-protein mixture.

The guide nucleic acid and the editor protein may be delivered or introduced into the subject in the form of a guide nucleic acid-editor protein complex. For example, the guide nucleic acid may be DNA, RNA, or a mixture thereof. The editor protein may be in the form of peptide, polypeptide, or protein. As an example, in the case of the guide nucleic acid and the editor protein, a guide nucleic acid in the form of RNA and an editor protein in the form of a protein may be delivered or introduced into the subject in the form of a guide nucleic acid-editor protein complex, that is, ribonucleoprotein (RNP).

In addition, the present disclosure provides a method of preparing cells for intravascular transplantation having hemocompatibility, including: (1) introducing the composition for preparing cells for intravascular transplantation having hemocompatibility into isolated mammalian cells or isolated stem cells; and (2) editing the F3 gene to reduce or suppress the expression or activity of CD142 by generating indels in the target sequence of the F3 gene located in the genome of the isolated mammalian cells or isolated stem cells.

At this time, the "introduction" may be performed by one or more means selected from electroporation, lipofection, microinjection, gene gun, liposomes, positive liposomes, plasmids, viral vectors, nanoparticles, protein translocation domain (PTD) Methods, immunoliposomes, polycations or lipids: nucleic acid conjugates, naked DNA, artificial virions, and preparation-enhanced uptake methods of DNA, but is not limited thereto.

In one embodiment of the present disclosure, the composition for producing cells for intravascular transplantation having hemocompatibility described above can be introduced into isolated mammalian cells or isolated stem cells by electroporation to produce cells for intravascular transplantation having hemocompatibility.

In one embodiment of the present disclosure, a CRISPR/Cas9 complex including Streptococcus pyogenes-derived Cas9 protein and guide RNA that may target the target sequence of the F3 gene is contacted with the F3 gene located within the genome of the mammalian cell, thereby generating an indel within the target sequence.

In one embodiment of the present disclosure, the target sequence may be the sequence of SEQ ID NO: 1.

The hemocompatible artificially engineered intravascular transplantation cells of the present disclosure may be used as a cell therapeutic agent for vascular administration and may be used to treat various diseases. For example, the stem cells may be used for heart disease, gastroduodenal disease, Small and large intestine diseases, liver disease, biliary tract disease, pancreas disease, kidney disease, liver disease, lung disease, mediastinal disease, diaphragm disease, pleural disease, peritoneal disease, neurological disease, central nervous system (CNS) disorder, peripheral arterial disease, and peripheral venous disease. Specific diseases may include, for example, liver diseases such as autoimmune hepatitis, fulminant hepatitis, chronic hepatitis, viral hepatitis, alcoholic hepatitis, nonalcoholic fatty liver disease (NAFLD), and nonalcoholic steatohepatitis (NASH), nonalcoholic fatty liver (NAFL), liver fibrosis, cirrhosis, liver cancer, fatty liver, drug-induced allergic liver disease, hemosiderosis, hemochromatosis, Wilson's disease, primary biliary cirrhosis (PBC), primary sclerosing cholangitis (PSC), biliary atresia, liver abscess, chronic active hepatitis, and chronic persistent hepatitis; heart diseases such as myocardial infarction, heart failure, arrhythmia, palpitations, cardiomyopathy, ischemic cardiomyopathy, angina pectoris, congenital heart disease, valvular disease, myocarditis, familial hypertrophic cardiomyopathy, dilated cardiomyopathy, acute coronary syndrome, arteriosclerosis, and restenosis; gastroduodenal diseases such as acute gastritis, chronic gastritis, gastroduodenal ulcer, stomach cancer, and duodenal cancer; Small and large intestine diseases such as ischemic enteritis, inflammatory bowel disease, ulcerative colitis, Crohn's disease, simple ulcer, intestinal Behcet's disease, small intestine cancer, and colon cancer; biliary duct diseases such as acute cholecystitis, acute cholangitis, chronic cholecystitis, cholangiocarcinoma, and gallbladder cancer; pancreatic diseases such as acute pancreatitis, chronic pancreatitis, and pancreatic cancer; kidney diseases such as acute nephritis, chronic nephritis, acute renal failure, and chronic renal failure; lung diseases, such as pneumonia, emphysema, pulmonary fibrosis, interstitial pneumonia, idiopathic interstitial pneumonia, exfoliative interstitial pneumonia, acute interstitial pneumonia, non-specific interstitial pneumonia, drug-induced lung disease, eosinophilic lung disease, pulmonary hypertension, pulmonary tuberculosis, sequelae of pulmonary tuberculosis, acute respiratory distress syndrome, cystic fibrosis, chronic obstructive pulmonary disease, pulmonary embolism, pulmonary abscess, pneumoconiosis, aspiration pneumonia, pulmonary fibrosis, acute upper respiratory tract infection, chronic lower respiratory tract infection, pneumothorax, alveolar epithelial damage disease, lymphangioleiomyoma, lymphatic interstitial pneumonia, alveolar proteinosis, and pulmonary Langerhans cell granulomatosis; mediastinal diseases such as mediastinal tumor, mediastinal cystic disease, and mediastinitis; diaphragmatic disease such as diaphragmatic hernia; pleural diseases such as pleurisy, empyema, pleural tumor, cancerous pleurisy, and pleural mesothelioma; peritoneal diseases such as peritonitis and peritoneal tumor; neurological diseases such as cerebral palsy syndrome, including pediatric cerebral palsy, aseptic meningitis, Guillain-Barre syndrome, amyotrophic lateral sclerosis (ALS), myasthenia gravis, mononeuropathy, polyneuropathy, spinal muscular atrophy, spinal disorder, acute, transverse myelitis, spinal cord infarction (ischemic myelopathy), intracranial tumor, and spinal tumor; CNS disorders such as Alzheimer's disease, cognitive impairment, stroke, multiple sclerosis, and Parkinson's disease; peripheral artery diseases such as fibromuscular dysplasia, peripheral artery disease (PAD), occlusive thromboangiitis (Buerger's disease), and Kawasaki disease (KD); peripheral venous diseases such as deep vein thrombosis, chronic venous insufficiency, postphlebitis syndrome, and superficial vein thrombosis; and immune dysfunction such as graft versus host Disease (GVHD), secondary immune deficiency, primary immunodeficiency disease, B cell deficiency, T cell deficiency, B and T cell combined deficiency, phagocyte deficiency, complement deficiency.

The term "for vascular administration" refers to delivery into the vascular system of a patient. As an example, it may be administration into a blood vessel considered to be a vein, or administration into a blood vessel considered to be an artery. Veins may include internal jugular vein, peripheral vein, coronary vein, hepatic vein, portal vein, great saphenous vein, pulmonary vein, superior vena cava, inferior vena cava, gastric vein, splenic vein, inferior mesenteric vein, superior mesenteric vein, cephalic vein, and/or femoral vein, but are not limited thereto. Arteries may include coronary artery, pulmonary artery, splenic artery,brachial artery, internal carotid artery, aortic arches, femoral artery, peripheral artery, and/or ciliary artery, but are not limited thereto. It may be delivered via the hepatic portal vein, umbilical vein, arteriole or capillary, or to the hepatic portal vein, umbilical vein, arteriole or capillary.

In one embodiment of the present disclosure, the artificially engineered intravascular transplantation cells of the present disclosure may be used as a composition for cell transplantation or regeneration of living tissue to restore damaged cells or tissues.

In one embodiment of the present disclosure, the biological tissue may be a damaged tissue selected from a tissue in which ulcers or bedsores have formed, brain tissue damaged by cell degeneration, brain tissue defected by surgical manipulation, brain tissue damaged by traumatic brain disease, brain tissue damaged by inflammatory brain disease, damaged bone tissue, damaged periodontal tissue, tissue damaged by central nervous system disease, and a tissue damaged by intractable dermatitis, and biological tissue regeneration may be restoration of damaged tissue, epidermal regeneration, reproduction of glands or hair follicles, microvascular formation in dermal tissue, wound healing, correction of soft tissue defects, bone healing, or bone regeneration, cartilage regeneration, or the like, but is not limited thereto.

In one embodiment of the present invention, the composition for cell transplantation or biological tissue regeneration may be a cell therapeutic composition, a gene therapeutic composition, a tissue engineering therapeutic composition, an immunotherapeutic composition, or a composition for the prevention or treatment of cancer.

As used herein, "cell therapeutic agent" refers to a medicine (US FDA regulations) used for the purposes of treatment, diagnosis, and prevention with cells and tissues prepared through isolation, culture, and special engineering from an individual, and refers to a medicine used for treatment, diagnosis, and prevention purposes through a series of actions such as changing the biological characteristics of cells by ex vivo proliferation selection or other methods of living autologous, allogeneic, or xenogeneic cells to restore cell or tissue function.

As used herein, "gene therapeutic agent" refers to a medicine administered to affect the expression of genetic material and containing cells into which genetic material has been modified or introduced.

The route of administration of a composition for cell transplantation or biological tissue regeneration of the present disclosure may be administered via any general route as long as the desired tissue may be reached. The route may be administered parenterally, for example, intraperitoneally, intravenously, intramuscularly, subcutaneously, or intradermally, but is not limited thereto.

The composition for cell transplantation or biological tissue regeneration may be formulated in a suitable form with a pharmaceutical carrier commonly used in cell therapy. The term "pharmaceutically acceptable" refers to a composition that is physiologically acceptable and does not usually cause allergic responses such as gastrointestinal disorders, dizziness, or similar responses when administered to humans. Pharmaceutically acceptable carriers include, for example, water, suitable oils, saline solutions, carriers for parenteral administration such as aqueous glucose and glycol, and may further include stabilizers and preservatives. The suitable stabilizer include antioxidants such as sodium bisulfite, sodium sulfite, or ascorbic acid. Suitable preservative include benzalkonium chloride, methylparaben or propyl-paraben, and chlorobutanol. As for other pharmaceutically acceptable carriers, those described in the following literature may be referred to (Remington's Pharmaceutical Sciences, 19th ed., Mack Publishing Company, Easton, PA, 1995). In addition, the composition may be administered by any device that allows the cell therapeutic agent to move to target cells.

The composition for cell transplantation or biological tissue regeneration of the present disclosure may include a therapeutically effective amount of cells for the treatment of disease. The term "therapeutically effective amount" refers to an amount of an active ingredient or pharmaceutical composition that induces a biological or medical response in a tissue system, animal, or human, as believed by a researcher, veterinarian, physician or other clinician, and includes an amount that lead to alleviation of the symptoms of the disease or disorder being treated.

It is obvious to those skilled in the art that the cells included in the composition of the present disclosure will vary depending on a desired effect. Therefore, an optimal cell content may be easily determined by those skilled in the art, and may be adjusted depending on a variety of factors, including the type of disease, the severity of the disease, the content of other ingredients contained in the composition, the type of formulation, and the patient's age, weight, general health, gender and diet, administration time, administration route and secretion rate of the composition, treatment period, and concurrently used medications. Considering all of the above factors, it is important to include a minimum amount of the maximum effect without side effects. For example, the daily dosage of the stem cells of the present disclosure is 1.0×10⁵ to 1.0×10³⁰ cells/kg of body weight, preferably 1.0×10¹⁰ to 1.0×10²⁰ cells/kg of body weight, and may be administered once or in several divided doses. However, it should be understood that the actual dosage of the active ingredient should be determined in light of various related factors such as the disease to be treated, the severity of the disease, the route of administration, the patient's weight, age, and gender, and therefore, the dosage does not limit the scope of the present disclosure in any way.

The present disclosure provides a treatment method including administering to mammals a therapeutically effective amount of the artificially engineered intravascular transplantation cells. As used herein, the term "mammal" refers to a mammal that is the subject of treatment, observation or experiment, preferably a human.

The present disclosure also includes a pharmaceutical composition for preventing or treating liver disease, including a therapeutically effective amount of the artificially engineered intravascular transplantation cells as an active ingredient.

The composition of the present disclosure may be administered orally or parenterally, including inhalation, intravascular, intraperitoneal, subcutaneous, rectal, and topical administration. In one embodiment of the present disclosure, the composition may be administered via the hepatic portal vein, umbilical vein, or splenic artery.

The present disclosure also provides a method for preventing or treating liver disease, including administering to mammals a therapeutically effective amount of the artificially engineered intravascular transplantation cells. As used herein, the term "mammal" refers to a mammal that is the subject of treatment, observation or experiment, preferably a human.

The present disclosure also provides a use of the artificially engineered intravascular transplantation cells for use in the manufacture of a medicament for the prevention or treatment of liver disease in mammals.

In one embodiment of the present disclosure, the liver disease includes cirrhosis; acute-on-chronic liver failure (ACLF); drug- or intoxication-induced liver failure; congenital metabolic liver disease; Crigler-Najjar syndrome type 1; familial hypercholesterolemia; factor VII deficiency; factor VIII deficiency (hemophilia A); phenylketonuria (PKU); glycogen storage disease type I; infantile Refsum disease; progressive familial intrahepatic cholestasis type 2; hereditary tyrosinemia type 1; urea cycle defect; acute liver failure; acute drug-induced liver failure; virus-induced acute liver failure; idiopathic acute liver failure; mushroom intoxication-induced acute liver failure; postoperative acute liver failure; acute liver failure due to acute fatty liver of pregnancy; alcoholic hepatitis; or hepatic encephalopathy.

The present disclosure also includes a pharmaceutical composition for preventing or treating diabetes, including a therapeutically effective amount of the artificially engineered intravascular transplantation cells as an active ingredient.

The composition of the present disclosure may be administered orally or parenterally, including inhalation, intravascular, intraperitoneal, subcutaneous, rectal, and topical administration. In one embodiment of the present disclosure, the composition may be administered via the hepatic portal vein.

The present disclosure also provides a method for preventing or treating diabetes, including administering to mammals a therapeutically effective amount of the artificially engineered intravascular transplantation cells.

The present disclosure also provides a use of the artificially engineered intravascular transplantation cells for use in the manufacture of a medicament for the prevention or treatment of diabetes in mammals.

In one embodiment of the present disclosure, the diabetes includes type 1 diabetes.

Hereinafter, the present disclosure will be described in more detail through examples. These examples are only for illustrating the present disclosure in more detail, and it will be apparent to those skilled in the art that the scope of the present disclosure is not limited by these examples.

### Example 1: F3 knock-out cell production

### Cell culture

iPSCs were cultured according to the manufacturer's manual. The cells were cultured using TeSR^{™}-E8^{™} (STEMCELL Technology) medium in a culture dish coated with Vitronectin XF^{™} (STEMCELL Technology) for 1 hour at room temperature.

### CD142 Gene editing cell production

RNP complexes were introduced into cultured cells by electroporation using a 4D-Nucleofector (Lonza). Specifically, the RNP complex was formed by mixing 4 ug of Cas9 protein and 4 ug of in vitro transcribed sgRNA (prepared according to the manufacturer's protocol using T7 polymerase (New England BioLabs)), and the mixture was incubated for 10 min at room temperature. The above RNP complexes were electroporated using the nucleofector program CA-137 together with 1x10⁶ iPSCs treated with 20 ul Primary P3 buffer. As a result, iPSCs with knocked out CD142 gene (CD142#33 KO iPSCs) were obtained. At this time, a guide RNA capable of targeting the target sequence 5'-TCTGGGGAGTTCTCATACAGAGG-3' (underlined: PAM sequence) (SEQ ID NO: 1) of CD142#33 was synthesized and introduced into iPSCs, and the indel efficiency of the target sequence was identified using a targeted deep sequencing method.

### Targeted deep sequencing

Genomic DNA (gDNA) was extracted from the obtained CD142#33 KO iPSC using a blood genomic DNA extraction kit (Favorgen) according to the manufacturer's protocol. To amplify a target region, 100 ng of genomic DNA (gDNA) was amplified using Phusion high-hidelity DNA polymerase PCR Polymerase (NEB). For deep sequencing library generation, amplicons were amplified once more using TruSeq HT dual index primers (Illumina, San Diego, CA, USA). Paired-end sequencing was performed using the Illumina Miniseq System, and the indel frequency was calculated at 'http://www.rgenome.net/'. The primer sequences for each target sequence used for targeted deep sequencing are shown in Table 1.

**[Table 1]**

| Classification | Forward primer (5'-3') | Reverse primer (5'-3') |
|---|---|---|
| sgCD142#33 | gcactaagtcaggagattgg | gttcagacgtttctaacaag |
| | (SEQ ID NO: 2) | (SEQ ID NO: 3) |

FIG. 1 is a graph showing the results of targeted deep sequencing of normal iPSCs and iPSCs that underwent gene editing of the target sequence (SEQ ID NO: 1) of CD142#33. As shown in the targeting deep sequencing results in FIG. 1, 98% indels were identified in iPSCs that underwent gene editing with the target sequence of CD142#33 (SEQ ID NO: 1).

### Pancreatic lineage cells differentiation

iPSCs with knocked out CD142 gene obtained above (CD142#33 KO iPSCs) were differentiated into pancreatic lineage cells. At this time, pancreatic lineage cell differentiation was performed according to the STEMCELL Technology^{™} protocol (catalog #05120) (https://www.stemcell.com/products/stemdiff-pancreaticprogenitor-kit.html#section-protocols-and-documentation), which differentiates into four stages: 1) terminal endoderm, 2) primitive intestine, 3) posterior full-length endoderm, and 4) pancreatic lineage cells. Differentiated cells were identified by expression of key markers of pancreatic lineage cells.

### Hepatocyte differentiation

iPSCs with knocked out CD142 gene obtained above (CD142#33 KO iPSCs) were differentiated into hepatocytes. At this time, hepatocyte differentiation was performed according to the Takara Bio protocol (catalog Y30050) (https://www.takarabio.com/products/stem-cell-research/media-and-supplements/stem-cell-differentiation-media-and-kits/hepatocyte-differentiation-kit). Differentiated cells were identified by expression of key markers of hepatocytes.

### Experimental Example 1: Identification of differentiation markers of pancreatic lineage cells derived from induced pluripotent stem cells (iPSCs)

To confirm whether iPSC cells were successfully differentiated into pancreatic lineage cells, markers that were particularly highly expressed in pancreatic lineage cells during differentiation were identified using qRT-PCR. The markers used in the experiment were pancreatic and duodenal homeobox 1 (PDX-1), NK6 Homeobox 1 (NKX6.1), and SRY-Box Transcription Factor 9 (SOX9).

mRNA was extracted from iPSCs using the RNeasy mini kit (Qiagen) according to the manufacturer's protocol. Thereafter, 1 µg of mRNA was reverse transcribed using a cDNA reverse transcription kit (Thermo Fisher Scientific). qRT-PCR was performed using PowerUp^{™} SYBR^{™} Green Master Mix using QuantStudio 3 (Thermo Fisher Scientific) according to the manufacturer's protocol. Gene expression levels were calculated using CT values, and GAPDH was used as an endogenous control. The results are shown in FIG. 2. Both the control group (normal iPSC), which is normal iPSC-derived pancreatic lineage cells, and the experimental group (iPSC-CD142#33), which is iPSC-derived pancreatic lineage cells with knocked out CD142 gene, showed an increase in marker expression on day 14 (D14) of differentiation, confirming that differentiation into pancreatic lineage cells was successful (N=4).

### Experimental Example 2: Identification of differentiation markers of liver cells derived from induced pluripotent stem cells (iPSCs)

To confirm whether iPSC cells were successfully differentiated into liver cells, markers that were particularly highly expressed in liver cells during differentiation were identified using qRT-PCR using the same method as in Example 1. The markers used in the experiment were hepatocyte nuclear factor 4 alpha (HNF4α), albumin (ALB), and alpha-fetoprotein (AFP). The results are shown in FIG. 3. As shown in FIG. 3, both the control group (normal iPSC), which is normal iPSC-derived liver cells, the experimental group (iPSC-CD142#33), which are iPSC-derived liver cells with knocked out CD142 gene, showed an increase in marker expression on day 40 (D40) of differentiation, confirming that differentiation into hepatocytes was successful.

### Enzyme-linked immunosorbent assay (ELSIA)

To confirm whether iPSC cells were successfully differentiated into liver cells, albumin (ALB), a marker that is particularly highly expressed in liver cells during differentiation, was identified using ELISA.

Albumin was measured using the cell lysate and cell culture supernatant of iPSC-derived liver cells using the Human Albumin EIA Kit (TaKaRa (MK132)). The experiment was preformed according to the protocol provided by the kit manufacturer. Absorbance was measured at a wavelength of 405 nm using a Multiskan^{™} FC Microplate Photometer (Thermo Scientific^{™} (51119000)) machine. The results are shown in FIG. 4.

As shown in FIG. 4, both the control group (normal iPSC), which is normal iPSC-derived liver cells, the experimental group (iPSC-CD142#33), which are iPSC-derived liver cells with knocked out CD142 gene, showed an increase in marker expression on day 40 (D40) of differentiation, confirming that differentiation into hepatocytes was successful.

### Experimental Example 3: Identification of CD142 gene knock-out in pancreatic lineage cells derived from induced pluripotent stem cell (iPSC)

### qRT-PCR

Knock-out (KO) of CD142 in iPSC-derived pancreatic lineage cells was identified by analysis of mRNA levels of the CD142 gene using qRT-PCR.

mRNA was extracted from iPSC-derived pancreatic lineage cells using the RNeasy mini kit (Qiagen) according to the manufacturer's protocol. Thereafter, 1 µg of mRNA was reverse transcribed using a cDNA reverse transcription kit (Thermo Fisher Scientific). qRT-PCR was performed using PowerUp^{™} SYBR^{™} Green Master Mix using QuantStudio 3 (Thermo Fisher Scientific) according to the manufacturer's protocol. Gene expression levels were calculated using CT values, and GAPDH was used as an endogenous control. The results are shown in FIG. 5. As shown in FIG. 5, on day 14 (D14) of differentiation, the CD142 mRNA level was decreased in the experimental group (iPSC-CD142#33), which is iPSC-derived pancreatic lineage cells in which the CD142 gene was knocked out, compared the control group (normal iPSC), which is normal iPSC-derived pancreatic lineage cells (N=4).

### Flow cytometer analysis (FACS)

CD142 expression cell level was analyzed using FACS to confirm that CD142 was knocked out (KO) in iPSC cell-derived pancreatic lineage cells. Cultured cells were washed twice with phosphate buffer saline (PBS), then cells were removed using 0.05% trypsin-EDTA and centrifuged at 1,000 rpm for 5 minutes. Cells were suspended in 100 ul of FACS staining buffer, mixed with antibodies, reacted at 4 °C for 1 h, washed twice with PBS, and suspended in 500 ul of PBS for FACS analysis. The results are shown in FIG. 6.

Referring to FIG. 6, on day 40 (D40) of differentiation, the percentage of cells showing CD142 expression (CD142 positive cell population, %) decreased in the experimental group (iPSC-CD142#33), which is iPSC-derived pancreatic lineage cells in which the CD142 gene was knocked out, compared to the control group (normal iPSC), which is normal iPSC-derived pancreatic lineage cells.

### Experimental Example 4: Identification of CD142 gene knock-out in liver cells derived from induced pluripotent stem cell (iPSC)

### qRT-PCR

As described above, knock-out (KO) of CD142 in iPSC-derived liver cells was identified by analysis of mRNA levels of the CD142 gene using qRT-PCR. The results are shown in FIG. 7. As shown in FIG. 7, on day 40 (D40) of differentiation, the CD142 mRNA level was decreased in the experimental group (iPSC-CD142#33), which is iPSC-derived liver cells in which the CD142 gene was knocked out, compared the control group (normal iPSC), which is normal iPSC-derived liver cells.

### FACS

As described above, CD142 expression cell level was analyzed using FACS to confirm that CD142 was knocked out (KO) in iPSC cell-derived liver cells. The results are shown in FIG. 8.

Referring to FIG. 8, on day 40 (D40) of differentiation, the percentage of cells showing CD142 expression (CD142 positive cell population, %) decreased in the experimental group (iPSC-CD142#33), which is iPSC-derived liver cells in which the CD142 gene was knocked out, compared to the control group (normal iPSC), which is normal iPSC-derived liver cells.

### Example 5: Identification of CD142 protein levels in induced pluripotent stem cell (iPSC)-derived pancreatic lineage cells and hepatocytes

### ELSIA

The amount of CD142 protein produced by knocking out (KO) CD142 in iPSC-derived pancreatic lineage cells and iPSC-derived liver cells was analyzed using ELISA experiment using cell lysates.

CD142 was measured using cell lysates and cell culture supernatants of iPSC-derived pancreatic lineage cells and liver cells using the Tissue Factor Activity Assay Kit (Human, Colorimetric, abcam (ab108906)). The experiment was preformed according to the protocol provided by the kit manufacturer. Absorbance was measured at a wavelength of 405 nm using a Multiskan^{™} FC Microplate Photometer (Thermo Scientific^{™} (51119000)) machine. The results are shown in FIGS. 9 and 10.

As shown in FIG. 9, on day 14 (D14) of differentiation, it can be confirmed that the experimental group (iPSC-CD142#33), which is iPSC-derived pancreatic lineage cells with the knocked out CD142 gene showed a decrease in the amount of CD142 protein in the cell lysate compared to the control group (normal iPSC), which is normal iPSC-derived pancreatic lineage cells (N=4).

Referring to FIG. 10, it can be confirmed that, on day 40 (D40) of differentiation, the experimental group (iPSC-CD142#33), which are iPSC-derived liver cells with the knocked out CD142 gene also showed a decrease in the amount of CD142 protein in the cell lysates compared to the control group (normal iPSC), which is normal iPSC-derived liver cells (N=4).

## Claims

1. Artificially engineered intravascular transplantation cells, which are artificially engineered mammalian cells comprising an artificially engineered F3 gene,
wherein the artificially engineered F3 gene is different from the F3 gene sequence of wild-type mammalian cells,
the artificially engineered F3 gene includes one or more indels in a nucleic acid sequence, and
an expression level of CD142 on the surface of the artificially engineered mammalian cells is reduced compared to that of the wild-type mammalian cells.

2. The artificially engineered intravascular transplantation cells of claim 1, wherein in the artificially engineered intravascular transplantation cells, an mRNA transcribed from the artificially engineered F3 gene has a lower mRNA expression level or a different sequence compared to the mRNA expression level transcribed from the F3 gene of the wild-type mammalian cells.

3. The artificially engineered intravascular transplantation cells of claim 1, wherein the indel is located within a protospacer-adjacent motif (PAM) sequence in a first, second, third, fourth or sixth exon region of the F3 gene, or within a contiguous sequence of 5 to 50 nucleotides adjacent to the 5' or 3' end of the PAM sequence.

4. The artificially engineered intravascular transplantation cells of claim 1, wherein the mammalian cells are selected from the group consisting of primary hepatocytes, primary pancreatic beta cells, primary pancreatic islet cells, pancreatic progenitor cells, pluripotent stem cell (PSC)-derived hepatocytes, PSC-derived pancreatic beta cells, PSC-derived pancreatic islet cells, PSC-derived pancreatic lineage cells, PSC-derived pancreatic progenitor cells, PSC-derived pancreatic organoids, PSC-derived pancreatic liver organoids, induced PSC(iPSC)-derived hepatocytes, iPSC-derived pancreatic beta cells, iPSC-derived pancreatic islet cells, iPSC-derived pancreatic lineage cells, iPSC-derived pancreatic progenitor cells, iPSC-derived pancreatic organoids, iPSC-derived pancreatic liver organoids, induced hepatocytes (iHeps) from somatic cells by compound or gene manipulation, induced islet cells from somatic cells by compound or gene manipulation, or induced pancreatic beta cells from somatic cells by compound or gene manipulation.

5. The artificially engineered intravascular transplantation cells of claim 1, wherein the sequence of the artificially engineered F3 gene does not include the sequence of SEQ ID NO: 1.

6. Artificially engineered intravascular transplantation cells, which are cells differentiated or derived from artificially engineered stem cells comprising an artificially engineered F3 gene,
wherein the artificially engineered F3 gene is different from the F3 gene sequence of cells differentiated or derived from wild-type stem cells,
the artificially engineered F3 gene includes one or more indels in a nucleic acid sequence, and
an expression level of CD142 on the surface of cells differentiated or derived from the artificially engineered stem cells is reduced compared to cells differentiated or derived from the wild-type stem cells.

7. The artificially engineered intravascular transplantation cells of claim 6, wherein the stem cells are induced pluripotent stem cells, embryonic stem cells, somatic cell nuclear transfer-PSC, or adult stem cells.

8. The artificially engineered intravascular transplantation cells of claim 6, wherein the cells differentiated or derived from the artificially engineered stem cells are pancreatic beta cells, hepatocytes, pancreatic progenitor cells, pancreatic islet cells, pancreatic lineage cells, pancreatic organoids, or pancreatic liver organoids.

9. A composition for preparing cells for intravascular transplantation having hemocompatibility, the composition comprising a guide nucleic acid comprising a guide sequence capable of targeting a target sequence of an F3 gene of mammalian cells or stem cells; and an editor protein or a nucleic acid encoding the same.

10. The composition of claim 9, wherein the composition includes the editor protein and the guide nucleic acid in the form of ribonucleoprotein (RNP).

11. The composition of claim 9, wherein the composition includes a nucleic acid encoding the editor protein and a nucleic acid encoding the guide nucleic acid in the form of one or more vectors.

12. The composition of claim 11, wherein the vector is selected from the group consisting of plasmid, retrovirus, lentivirus, adenovirus, adeno-associated virus, vaccinia virus, poxvirus, and herpes simplex virus.

13. A preparation method for cells for intravascular transplantation having hemocompatibility, the method comprising:
(1) introducing into isolated mammalian cells or isolated stem cells a composition for producing cells for intravascular transplantation having hemocompatibility, including a guide nucleic acid capable of targeting a target sequence of F3 gene or a nucleic acid encoding the same; and an editor protein or a nucleic acid encoding the same into isolated intravascular transplantation cells; and
(2) editing the F3 gene to reduce or suppress the expression or activity of CD142 by generating indels in the target sequence of the F3 gene located in the genome of the isolated mammalian cells or isolated stem cells.

14. The method of claim 13, wherein the composition includes a nucleic acid encoding the editor protein and a nucleic acid encoding the guide sequence in the form of one or more vectors.

15. The method of claim 13, wherein a CRISPR/Cas9 complex including Streptococcus pyogenes-derived Cas9 protein and guide RNA that may target the target sequence of the F3 gene is contacted with the F3 gene located within the genome of the isolated mammalian cells, thereby generating indels within the target sequence.

16. A cell therapeutic agent for vascular administration comprising the artificially engineered intravascular transplantation cells of any one of claims 1 to 8 as an active ingredient.

17. A pharmaceutical composition for preventing or treating liver disease, the pharmaceutical composition comprising the artificially engineered intravascular transplantation cells of any one of claims 1 to 8 as an active ingredient.

18. The pharmaceutical composition of claim 17, wherein the liver disease is cirrhosis; acute-on-chronic liver failure (ACLF); drug- or intoxication-induced liver failure; congenital metabolic liver disease; Crigler-Najjar syndrome type 1; familial hypercholesterolemia; factor VII deficiency; factor VIII deficiency (hemophilia A); phenylketonuria (PKU); glycogen storage disease type I; infantile Refsum disease; progressive familial intrahepatic cholestasis type 2; hereditary tyrosinemia type 1; urea cycle defect; acute liver failure; acute drug-induced liver failure; virus-induced acute liver failure; idiopathic acute liver failure; mushroom intoxication-induced acute liver failure; postoperative acute liver failure; acute liver failure due to acute fatty liver of pregnancy; alcoholic hepatitis; or hepatic encephalopathy.

19. The pharmaceutical composition of claim 17, wherein the pharmaceutical composition is administered through hepatic portal vein, umbilical vein, or splenic artery.

20. A pharmaceutical composition for preventing or treating diabetes comprising the artificially engineered intravascular transplantation cells of any one of claims 1 to 8 as an active ingredient.

21. The pharmaceutical composition of claim 20, wherein the diabetes is type 1 diabetes.

22. The pharmaceutical composition of claim 20, wherein the pharmaceutical composition is administered through the hepatic portal vein.

23. A composition for cell transplantation or biological tissue regeneration, the composition comprising the artificially engineered intravascular transplantation cells of any one of claims 1 to 8 as an active ingredient.

24. The composition of claim 23, wherein the composition for cell transplantation or biological tissue regeneration is a cell therapeutic composition, a gene therapeutic composition, a tissue engineering therapeutic composition, an immunotherapeutic composition, or a composition for the prevention or treatment of cancer.
